(19) Europäisches Patentamt
European Patent Office
Office européen des brevets

(11) **EP 4 225 255 B1**

(12) **EUROPEAN PATENT SPECIFICATION**

(45) Date of publication and mention
of the grant of the patent:
**27.08.2025 Bulletin 2025/35**

(21) Application number: **21782768.2**

(22) Date of filing: **07.10.2021**

(51) International Patent Classification (IPC):
*A61K 6/54* $^{(2020.01)}$     *A61K 6/69* $^{(2020.01)}$
*A61K 6/76* $^{(2020.01)}$

(52) Cooperative Patent Classification (CPC):
**A61K 6/54; A61K 6/69; A61K 6/76**

(86) International application number:
**PCT/EP2021/077676**

(87) International publication number:
**WO 2022/074110 (14.04.2022 Gazette 2022/15)**

(54) **AN ISOPRENE-BASED MATRIX COMPOSITION**

MATRIXZUSAMMENSETZUNG AUF ISOPRENBASIS

COMPOSITION DE MATRICE À BASE D'ISOPRÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2020 EP 20382886**

(43) Date of publication of application:
**16.08.2023 Bulletin 2023/33**

(73) Proprietor: **Universitat Internacional De
Catalunya
08017 Barcelona (ES)**

(72) Inventors:
• **PÉREZ ANTOÑANZAS, Román
08850 GAVÀ (ES)**
• **GIORDANO, Ana Barbara
08012 BARCELONA (ES)**
• **DURÁN-SINDREU TEROL, Fernando
08024 BARCELONA (ES)**

• **GONZÁLEZ SÁNCHEZ, José Antonio
08173 SANT CUGAT DEL VALLÈS (ES)**

(74) Representative: **ZBM Patents - Zea, Barlocci &
Markvardsen
Rambla de Catalunya, 123
08008 Barcelona (ES)**

(56) References cited:
**JP-A- 2016 098 202     US-A1- 2002 037 258
US-A1- 2008 085 948**

• **SAVARMAND SAEID ET AL: "Rheological
properties of concentrated aqueous silica
suspensions: Effects of pH and ions content",
JOURNAL OF RHEOLOGY, vol. 47, no. 5, 25
August 2003 (2003-08-25), US, pages 1133 - 1149,
XP055866330, ISSN: 0148-6055, DOI: 10.1122/
1.1603237**

**Description**

The present invention relates to the field of dentistry. In particular, to a composition comprising an isoprene-based matrix and sol-gel silica microspheres for use as root canal filling agent. It also relates to a proces for its preparation as well as to a process for applying them in a root canal.

**Background Art**

Root filling materials have a long history of successful application in dentistry, particularly in endodontology. Root canal treatment aims to remove the infected tissues (i.e.pulp, root apex and periapical bone) and to seal/cut off the communication between oral environment and periapical tissue occurring through the root canal using root canal fillers.

An ideal root canal filling material should be able to create an accurate 3 dimensional obturation of all the length of the endodontic space. Failures are related to the coronal-to-apical leakage due to unsatisfactory physical-chemical properties and to the presence of gaps, microchannels and porosities. A successful root canal treatment depends critically on the physical blockage of the root canal from invading bacteria originating in the oral cavity. Therefore, the root canal fillings must ensure impervious sealing and should be volumetrically stable, low soluble, low porous, biocompatible and able to positively interact with the periapical bone tissue.

The Japanese patent document JP2016098202 discloses a dental polymerizable composition comprising a (meth)acrylic polymerizable monomer, a hydroperoxide compound, a thiourea derivative, a transition metal compound, a softening agent (e) and a amorphous silica powder (commercially available as KE250) having a average particle diameter of 2.5 micrometers. However, this silica microspheres are not sol-gel silica microspheres. Further, And, it is also disclosed in the state of the art a dental composition containing an amorphous bioactive particulate material consisting essentially of calcium, silicon and oxygen (cf. US2002037258).

Thermoplastic polyisoprene based materials, such as gutapercha (introduced by Bowman in 1867 as the sole material for filling root canals) have been widely used due to the fact that they can be warmed to improve its adaptation to the complex root canal anatomy. For instance, the US2008085948 discloses an obturating and sealing dental root canals compositions comprising a mixture of gutta-percha and a hydraulic material containing calcium silicate, calcium aluminate, calcium phosphate, and calcium sulfate compounds are disclosed.

Unfortunately, heated gutapercha shrinks considerably upon cooling to body temperature. To compensate for this shrinkage, elaborate application techniques and a canal wall lining material (called sealer) has been used. Sealers may adhere with varying strength to filling agent (such as gutapercha) and the root canal wall. However, even the use of selaers, the application of a combination of a root canal filler and a sealer still results in microleakage.

In order to avoid these microleakage, it was recommended the use unique single self-adhesive root canal filler, which after application forms a monoblock system. In this sense, calcium silicate cements are widely used to fill teeth with open apices, for perforation repair and as root end filling materials after root end resection. These calcium silicate cements can be adhered to root dentine by forming a crystalline bond in a biochemical process termed biomineralization. However, root canals filled with such calcium silicatebased cements can never be retreated, a feature that is deemed disadvantage by most clinicians.

In order to solve this drawback, bioactive glass materials have been incorporated into matrix polymers of core root filling materials. These bioactive glasses have the ability to form a mineralized layer on their surface in a physiologic environment. In particular, the phosphate bioactive glasses are able of precipitating calcium phosphate (CaP) crystals at the material's surface. However, the phosphate glass still have some disadvantages such as a complex manufacturing process, low purity and homogeneity as weel as a limited antimicrobial effect which is another essential requirement of root canal filling agents.

In subsequent researchs, an antimicrobial nanometric flame-spraying (melt) bioactive glasses were incorporated into polyisoprene (the matrix polymer of gutapercha). This materials showed good immediate sealing properties when heated and applied into simulated root canals in resin blocks. Based on these results, a commercial product was developed containing bioactive glass of the 45S5 type (Bio-Gutta; smartodont IIc, Zurich, Switzerland). However, in the practise, clinicians apply this polyisoprene matrix containing nanometric melt bioactive glasses as root canal fillers in combination with root canal sealers, which suggest that microleakages still remain and a complete sealing of the root canal is not achieved without the use of the sealer.

Furthermore, as it is disclosed in the state of the art, the nanometric melt bioactive glasses prepared by flame spray synthesis is not homogeneously distributed within the polyisoprene matrix causing an agregation of the bioactive glasses on the surface of the material (cf. D. Mohnet al. "composites made of flame-sprayed bioactive glass 45S5 and polymers: bioactivity and inmediate sealing properties", International Endodontic journal, 2010, vol. 43, pages 1037-1046). Without being bound to any theory, it seems that the distribution of the nanoparticles mainly on the surface of the polyisoprene in form of agregates enhance the formation of hydroxiapatite on the surface of the material. These agregates allow strengthing the linkage between the bioactive glass and the dentine, but not the linkage between the bioactive glass and the polyisoprene matrix causing the formation of holes and the subsequent formation of microleakages and re-infections.

Thus, from what is known in the state of the art, there is still the need of providing a biocompatible antimicrobial self-adhesive root canal filler.

**Summary of Invention**

Inventors have found that the presence of sol-gel silica microspheres homogeneously distributed in a polymer of isoprene allows increasing the adhesion of the polyisoprene matrix to the surface of the dentine without the need of sealers. Without being bound to any theory, it seems that the homogeneous distribution of the sol-gel microspheres allows enhancing the mechanical properties of the polyisoprene matrix resulting in the formation of an uniform apatite network (monolithic-block), both on the surface but also inside the matrix, upon contact with the fisiological media. This homogeneous apatite's net avoids the formation of gaps and the subsequent re-infection and/or creation of new leaks results becoming in a highly biocompatible and self-adhesive composition.

As it is demonstrated in the experimental section, the mechanical resistance of the compositions of the invention has been increased, meanwhile the deformation degree by expansion or compression has been reduced. These mechanical changes are advantegeous because it means that the composition of the present invention does not modify its size even inside the root canal ensuring a total sealing of the root canal for a prolonged period of time, but reversible if desired.

Furthermore, the composition of the present invention are also advantegeous because have an intrinsic antibacterial activity due to the presence of polyisoprene matrix that is maintained even of the presence of the sol-gel silica microspheres. Advantageously, the intrinsec antibacterial activity can be promoted by loading or doping the polyisoprene matrix with further pharmaceutical active ingredients that can be release from the matrix in a prolonged/-controlled/modified release adding their pharmacological benefits more locally.

Thus, the first aspect of the invention refers to a composition comprising: a polyisoprene-based matrix; and sol-gel silica microspheres; wherein: the sol-gel silica microspheres are homogeneously distributed in the polyisoprene-based matrix. Particularly, a root canal filling composition comprising: a polyisoprene-based matrix; and sol-gel silica microspheres; wherein: the sol-gel silica microspheres are homogeneously distributed in the polyisoprene-based matrix; the polyisoprene-based matrix is selected from the group consisting of trans-polyisoprene, gutapercha, and mixtures thereof; and the sol-gel silica microspheres have a diameter from 5 $\mu$m to 500 $\mu$m measured by dynamic laser scattering.

The second aspect of the invention refers to a process for the preparation of the composition as defined in the first aspect of the invention, comprising: (i) mixing polyisoprene with sol-gel silica microspheres; and optionally, with one or more excipients or carriers; and optionally one or more solvents; (ii) drying the composition obtained in step (i); and (iii) optionally, molding the composition obtained in step (ii); and drying the composition thus obtained.

The third aspect of the invention refers to the composition of the first aspect of the invention for use in dentistry.

The fourth aspect of the invention refers to a process for filling a root canal comprising applying the composition of the first aspect of the invention.

**Brief Description of Drawings**

Fig. 1 shows the homogeneous distribution of the sol-gel silica microspheres in the polyisoprene matrix composition of the present invention.

Fig. 2 shows a Fourier Transformed Infrared Spectroscopy (FTIR) expressed as transmittance (%) versus wavenumber (cm-1) of the sol-gel silica polyisoprene matrix composition of the present invention (composition Ex.1; 10-SiMS-GP), gutapercha (GP) and sol-gel silica microspheres (SiMS).

Fig. 3 shows the mechanical properties of the sol-gel silica polyisoprene matrix composition of the present invention (Composition Ex.1: GP+MS in the black bar), and the commercial gutapercha (comGP in the grey bar). Fig.3A shows the yield stress expressed in MPa and Fig. 3B shows the elastic modulus expressed in MPa.

Fig. 4 shows the in vitro apatite forming ability of the composition of the present invention (composition Ex.1 in Fig. 4B) in comparison with the gutapercha (Fig. 4A) by the use of Scanning Electron Microscopy (SEM) pictures.

Fig. 5 shows the cell viability expressed in percentage (%) versus time expressed in days of commercial gutapercha (GP), the composition of the present invention (composition Ex.1: GP+MS), sol-gel silica microspheres (MS) and control (CT).

Fig 6. shows the sealing ability of the composition of the present invention (composition Ex.1: GP-MS) in comparison with the commercial gutapercha (GP) on dental training blocks.

Fig.7. shows the sealing ability of the composition of the present invention (composition Ex.1: GP-MS) in comparison with the commercial gutapercha (GP) on extracted teeth.

**Detailed description of the invention**

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present invention, any ranges given include both the lower and the upper end-points of the range. Ranges and values given, such as temperatures, times, and the like, should be considered approximate, unless specifically stated.

The terms "percentage (%) by weight", "weight/weight %" and "w/w %" have the same meaning and are used interchangeable. They refer to the weight of a component in relation to the total weight of the composition.

The terms "about" or "around" as used herein refers to a range of values ± 10% of a specified value. For example, the expression "about 6" or "around 6" includes ± 10% of 6, i.e. from 5.4 to 6.6.

As it is mentioned above, the first aspect of the invention refers to a composition comprises sol-gel silica microspheres homogeneously distributed in the polyisoprene-based matrix. Particularly, a root canal filling composition comprising: a polyisoprene-based matrix; and sol-gel silica microspheres; wherein: the sol-gel silica microspheres are homogeneously distributed in the polyisoprene-based matrix; the polyisoprene-based matrix is selected from the group consisting of trans-polyisoprene, gutapercha, and mixtures thereof; and the sol-gel silica microspheres have a diameter from 5 μm to 500 μm measured by dynamic laser scattering.

The terms "polyisoprene", "poly-isoprene", "polymers of isoprene" and "isoprene polymer" have the same meaning and are used interchangeable. The term polyisoprene refers to a polymer of isoprene (i.e. 2-methyl-1,3-butadiene) encompassing both natural and synthetic polyisoprene. Natural polyisoprene includes polyisoprene having a 1,4 bond in a cis form and polyisoprene having a 1,4 bond in a trans form. On the other hand, in addition to the above two types, synthetic polyisoprene includes polyisoprene bonded by 1,2 bonds or by 3,4 bonds.

The molecular weight and the degree of polymerization of the polyisoprene of the present invention are not particularly limited. For instance, the number average molecular weight of the polyisoprene of the present invention is from 500 to 10,000,000, more preferably 500 to 5,000,000, still more preferably 1,000 to 5,000,000, still more preferably 5,000 to 1,000,000. The number average molecular weight can be measured by any method known in the state of the art.

In an embodiment, the polyisoprene used in the present invention is obtained from natural products. In this case, any plant can be used as a natural product as long as it is a plant that produces polyisoprene. In general, polyisoprene produced by plants can be classified into three types because of its structure. That is, cis-type polyisoprene, trans-type polyisoprene, and polyisoprene in which cis-type and trans-type are mixed. Among natural polyisoprenes (isoprenoids), there are many plants that produce cis-type polyisoprene, and it is known that the number is over 2000 species. Specifically, as plants that produce cis-type polyisoprene, for example, plants of the family Mulaceae, Asteraceae, and Musselae are known, and Para rubber tree (Hevea brasiliensis) is particularly known. On the other hand, there are very few plants that produce trans-polyisoprene, and only a few types are known, including plants of the genus Eucommia, Palaquium, and Payena. Specifically, Eucommia ulmoides Oliver, Palaquim gutta and Mimusops balata are known as plants that produce trans-type polyisoprene. Further, as a plant that produces a mixture of cis-type polyisoprene and trans-type polyisoprene, sapodilla (Achras zapota) is known. Therefore, it is possible to obtain the polyisoprene of the present invention from the above-mentioned natural products. For example, in order to obtain trans-type polyisoprene, well-known trans-polyisoprene-producing plants such as Tochu, Guttapercanoki, Balata rubber, sapodilla can be used, and it is not yet known to produce trans-type polyisoprene. Since the number of cultivations is large, as the trans-type polyisoprene-producing plant used in the present invention, Tochu, Gutta Percan, and Balata rubber are preferable.

In an embodiment, the composition of the invention comprises polyisoprene selected from the group consisting of trans-polyisoprene, gutta-percha, and mixtures thereof.

In an embodiment, the composition of the invention comprises polyisoprene being trans-polyisoprene. The trans-polyisoprene used in the present invention is a polymer of isoprene being bonded mainly through trans-type 1,4 bonds, and may be obtained from nature or obtained by synthesis.

In an embodiment, the composition of the invention comprises polyisoprene being guttapercha. The terms "guta-percha", "gutapercha", "gutta", "guttapercha" and "gutta-percha" have the same meaning and are used interchangeable.

They refer to a thermoplastic substance mainly composed of trans-polyisoprene obtained from plant emulsion. It is known that gutta percha contains resinous esters in addition to trans-polyisoprene. As used herein, gutta percha includes, for example, thermoplastic materials obtained from the emulsions of Eucommia, Palaquium, Payena, Mimusops and Achras plants. Eucommia genus plants include Tochu, Paraquium genus plants include gutta percanoki, Mimusops genus Balata rubber, and Achras genus plants include sapodilla, but are not limited thereto. Gutapercha is commercially available in form of cones with the tradename Dentsply Maillefer® and it is composed of 20% trans-polyisoprene (TPI), 66% zinc oxide ($ZnO_2$), 11% barium sulphate ($BaSO_4$) and 3% wax.

The term "matrix" as used herein is defined as the combination of the polyisoprene and the sol-gel silica microspheres, and optionally the pharmaceutical active ingredients, excipients and carriers, wherein the microspheres are included to the polyisoprene by intermolecular forces such as electrostatic interactions, hydrogen bonding and van der Waal forces, amongst others.

As it is mentioned above, the first aspect of the invention refers to a composition comprises sol-gel silica microspheres homogeneously distributed in the polyisoprene. It seems that the homogenous distribution of the microspheres in combination with the nature of the microspheres are responsible of the enhanced mechanical and sealing properties as well as adhesion capability of the composition of the invention.

In an embodiment, the composition of the present invention comprises a sol-gel silica microspheres density from 0.011 to 5 mg per mm2. In an embodiment, the composition of the present invention comprises a sol-gel silica microspheres density from 0.02 to 2.5 mg per mm2. In an embodiment, the composition of the present invention comprises a sol-gel silica microspheres density from 0.04 to 1.25 mg per mm2. In an embodiment, the composition of the present invention comprises a sol-gel silica microspheres density from 0.08 to 0.75 mg per mm2. In an embodiment, the composition of the present invention comprises a sol-gel silica microspheres density from 0.15 to 0.50 mg per mm2. The measurement of the microspheres density is performed using any of the methods known in the state of the art. For the purpose of the invention, the microspheres density is measured by image analysis. For this purpose, different images were taken and analyzed with ImageJ, selecting random areas measuring 1 $mm^2$ and counting the number of microspheres within the different regions. The weight of the microspheres was calculated by weighting a random amount of microspheres and dividing the total weight by the number of counted microspheres. The density herein described was expressed as microsphere weight per square milimeter.

For the purpose of the invention, the term "sol-gel" or "solution sol-gel" or "SSG" have the same meaning and are used interchangeable. They refer to microspheres obtainable by a "sol-gel" process which is a chemical process that are used for the synthesis of single- or multiple-component materials, including glasses, in the form of thin solid films, ultrafine powders, high surface area porous materials, dense abrasive minerals, and continuous ceramic and glass fibres. In particular, a sol-gel process involves the preparation of one or more precursor mixtures (also called "sol"), which is converted into intermediate product (also called "gel") and thereof into a specified material by a process that may involve chemical reactions, product forming, gelification, drying, and/or curing.

The term "sol" refers to either a dispersion of colloidal particles of one phase in a fluid medium or a solution prepared by hydrolysis and polycondensation of metalorganic derivatives compounds or inorganic salts in alcoholic solution. And, the term "gel" refers to a material consisting of a three-dimensional network of a solid phase interwoven with an entrapped and immobilized continuous liquid phase.

The term "silica microspheres" refers to a plurality of polymeric spherical particles having a silica surface arranged in an interconnected three-dimensional lattice and with a diameter in the range from 5 μm to 500 μm. Particles with diameters below 0.1 μm are called nanospheres. In an embodiment, the sol-gel silica microspheres have a diameter from 10 μm to 400 μm. In an embodiment, the sol-gel silica microspheres have a diameter from 15 μm to 300 μm. In an embodiment, the sol-gel silica microspheres have a diameter from 10 μm to 200 μm. In an embodiment, the sol-gel silica microspheres have a diameter from 20 μm to 100 μm. This diameter is especially advantageous because it allows better homogenization as well as higher sites of contact with dentin. The diameter of the microspheres can be measured using any method known in the state of the art. For the purpose of the present invention the diameter of the microspheres is measured by optical analysis and dynamic laser scattering (cf. Ross Hallett "Particle size analysis by dynamic light scattering". Food Research International, 1994, vol. 27, issue 2, pp. 195-198)

In an embodiment, the composition of the present invention comprises sol-gel silica microspheres, which comprises a therapeutically effective amount of one or more pharmaceutical active ingredients.

In an embodiment, the composition of the invention comprises sol-gel silica microspheres obtainable by a process which comprises:

(a) providing a sol by mixing one or more silicon alkoxides with one or more acid catalysts;
(b) adjusting the pH of the sol obtained in step (a) from 4 to 5.5;
(b') optionally, adding the therapeutically effective amount of the one or more pharmaceutical active ingredient, and optionally stirring until having a homogeneous mixture within the sol;
(c) adding the sol obtained in step (b) or (b') to an oil phase, and stirring until having the microspheres; and

(d) optionally, isolating the microspheres obtained in step (c).

For the purposes of the invention the expressions "obtainable", "obtained" and equivalent expressions are used interchangeably, and in any case, the expression "obtainable" encompasses the expression "obtained".

The term "silicon alkoxide" refers to a compound characterized by a silicon central atom that forms covalent bonds with oxygen-carbon, namely silicon-oxygen-carbon bonds. For the purpose of the invention, the silicon alkoxide comprises from 4 to 56 carbon atoms.

In an embodiment, the composition of the invention comprises sol-gel silica microspheres obtainable by a process where in step (a) the silicon alkoxide is a compound of formula (I)

$$(OR^1)(OR^2)(OR^3)(OR^4)Si \qquad (I)$$

wherein:
each one of $R^1$, $R^2$, $R^3$ and $R^4$ are independently selected from the group consisting of substituted or un-substituted $(C_1-C_{14})$alkyl, $(C_5-C_6)$aryl, $(C_2-C_{14})$alkenyl and $(C_2-C_{14})$alkynyl group.

The term" alkyl" refers to a saturated straight, or branched hydrocarbon chain that contains the number of carbon atoms specified in the description or claims. Examples include, among others, the group methyl, ethyl, propyl, isopropyl, butyl, isobutyl, sec-butyl, tert-butyl, n-pentyl, and n-hexyl. The term "alkenyl" refers to a saturated straight, or branched alkyl chain that contains from 2 to 14 carbon atoms and one or more double bonds. Examples include, among others, ethenyl, 1-propen-1-yl, 1-propen-2-yl, 3-propen-1-yl, 1-buten-1-yl, 1-buten-2-yl, 3-buten-1-yl, 3-buten-2-yl, 2-buten-1-yl, 2-buten-2-yl, 2-methyl-1-propen-1-yl, 2-methyl-2-propen-1-yl, 1,3-butadien-1-yl, 1,3-butadien-2-yl, and 2-hexenyl. The term "alkynyl" refers to a saturated straight, or branched alkyl chain that contains from 2 to 14 carbon atoms and one or more triple bonds. Examples include, among others, ethynyl, 1-propynyl, 2-butynyl, 1,3-butadinyl, 4-pentynyl, and 1-hexynyl. The term "aryl" refers to a 5 to 6 membered ring, saturated, partially or totally unsaturated, optionally bridged or fused to a 5 to 6 membered ring; the members of the rings being independently selected from C, CH, $CH_2$, O, N, NH, and S; being one or more of the hydrogen atoms of the members optionally substituted by a radical selected from the group consisting of -$(C_1-C_6)$alkyl, -$(C_1-C_6)$alkyl-halogen, halogen, $(C_1-C_6)$alkyl-O-, $(C_1-C_6)$alkyl-CO-, $(C_1-C_6)$alkyl-O-CO-, -$NO_2$ and NC-. The term "halogen" refers to fluorine, chlorine, bromine and iodine.

In an embodiment, the composition of the invention comprises sol-gel silica microspheres obtainable by a process where in step (a) the silicon alkoxide is selected from the group consisting of tetramethyl orthosilicate (TMOS), tetrapropyl orthosilicate (TPOS), tetrabutyl orthosilicate (TBOS) and tetraethyl orthosilicate (commonly known as TEOS). In an embodiment, the composition of the invention comprises sol-gel silica microspheres obtainable by a process where in step (a) the silicon alkoxide is tetraethyl orthosilicate (commonly known as TEOS).

In an embodiment, the composition of the invention comprises sol-gel silica microspheres obtainable by a process wherein the pH of step (b) is from 4.5 to 5.5. In an embodiment, the composition of the invention comprises sol-gel silica microspheres obtainable by a process wherein the pH of step (b) is from 5.0 to 5.5.

In an embodiment, the composition of the invention comprises sol-gel silica microspheres obtainable by a process wherein in step (b) the acid catalyst is an inorganic acid selected from the group consisting of $H_2SO_4$, HCl, $HNO_3$, and a mixture thereof. In an embodiment, the composition of the invention comprises sol-gel silica microspheres obtainable by a process wherein in step (b) the acid catalyst is HCl.

In an embodiment, the composition of the invention comprises sol-gel silica microspheres obtainable by a process wherein in step (b) the acid catalyst is in form of an aqueous solution. In an embodiment, the composition of the invention comprises sol-gel silica microspheres obtainable by a process wherein in step (b) the acid catalyst is in form of an aqueous solution having a pH equal to or lower than 3.5; preferably the pH is from 1 to 3. In an embodiment, the composition of the invention comprises sol-gel silica microspheres obtainable by a process wherein in step (b) the acid catalyst is an aqueous solution of HCl having a pH equal to or lower than 3.5; particularly from 1 to 3.

In an embodiment, the composition of the invention comprises sol-gel silica microspheres obtainable by a process wherein the molar ratio between the water and silicon alkoxide is from 4 to 12. In an embodiment, the molar ratio between water and silicon alkoxide is 6 to 10. In an embodiment, the molar ratio between water and silicon alkoxide is 7 to 9.

In an embodiment, the composition of the invention comprises sol-gel silica microspheres obtainable by a process where: in step (a) the silicon alkoxide is selected from the group consisting of tetramethyl orthosilicate (TMOS), tetrapropyl orthosilicate (TPOS), tetrabutyl orthosilicate (TBOS) and tetraethyl orthosilicate (commonly known as TEOS), and in step (b) the acid catalyst is an inorganic acid selected from the group consisting of $H_2SO_4$, HCl, $HNO_3$, and a mixture thereof; particularly an aqueous solution thereof. In an embodiment, the composition of the invention comprises sol-gel silica microspheres obtainable by a process where: in step (a) the silicon alkoxide is tetraethyl orthosilicate (commonly known as TEOS), and in step (b) the acid catalyst is HCl; particularly an aqueous solution thereof.

In an embodiment, the composition of the invention comprises sol-gel silica microspheres obtainable by a process wherein step (b) is performed by the addition of an amount of one or more pH adjusting agents until having the required pH value. In an embodiment, the composition of the invention comprises sol-gel silica microspheres obtainable by a process wherein in step (b) the one or more pH adjusting agents is selected from ammonium hydroxide, alkaline or alkaline earth metal hydroxide, and a mixture thereof. In an embodiment, the composition of the invention comprises sol-gel silica microspheres obtainable by a process wherein in step (b) the one or more pH adjusting agents areselected from the group consisting of sodium hydroxyde, calcium hydroxyde, potassium hydroxyde and ammonium hydroxide. In an embodiment, the composition of the invention comprises sol-gel silica microspheres obtainable by a process wherein in step (b) the pH adjusting agent is ammonium hydroxide.

In an embodiment, the composition of the invention comprises sol-gel silica microspheres obtainable by a process wherein step (b') is performed, thereby the composition of the invention is a pharmaceutical composition and the sol-gel silica microspheres comprises a therapeutically effective amount of one or more pharmaceutical active ingredients.

In an embodiment, the composition of the invention comprises sol-gel silica microspheres obtainable by a process wherein in step (c) the oil phase comprises natural, synthetic or semisynthetic oils. The nature of the oil is not particularly limited. Examples of appropriate oils include, but not limited, to olive oil, sunflower oil, vaseline ol, palm oil and mixture thereof.

In an embodiment, the composition of the invention comprises sol-gel silica microspheres obtainable by a process which comprises:

(a) providing a sol by mixing tetraethyl orthosilicate with an aqueous solution of HCl;
(b) adjusting the pH of the sol obtained in step (a) from 4 to 5.5;
(b') optionally, adding the therapeutically effective amount of the one or more pharmaceutical active ingrediant, and optionally stirring until having a homogeneous mixture within the sol;
(c) adding the sol obtained in step (b) to an oil phase, and stirring until having the microspheres; and
(d) optionally, isolating the microspheres obtained in step (c).

In an embodiment, the composition of the invention comprises sol-gel silica microspheres obtainable by a process which comprises:

(a) providing a sol by mixing tetraethyl orthosilicate with an aqueous solution of HCl;
(b) adjusting the pH of the sol obtained in step (a) from 4 to 5.5 by the addition of ammounium hydroxide;
(b') optionally, adding the therapeutically effective amount of the one or more pharmaceutical active ingrediant, and stirring until having a homogeneous mixture;
(c) adding the sol obtained in step (b) to an oil phase, and stirring until having the microspheres; and
(d) optionally, isolating the microspheres obtained in step (c).

In an embodiment, the composition of the invention comprises sol-gel silica microspheres obtainable by a process which comprises:

(a) providing a sol by mixing tetraethyl orthosilicate with an aqueous solution of HCl;
(b) adjusting the pH of the sol obtained in step (a) from 4 to 5.5 by the addition of ammounium hydroxide;
(b') optionally, adding the therapeutically effective amount of the one or more pharmaceutical active ingrediant, and optionally stirring until having a homogeneous mixture within the sol;
(c) adding the sol obtained in step (b) to an olive oil phase, and stirring until having the microspheres; and
(d) optionally, isolating the microspheres obtained in step (c).

In an embodiment, the composition of the invention comprises sol-gel silica microspheres obtainable by a process comprising a isolation step (d). Commonly, the isolation step (d) may include removing the solvent such as water, for example, by one or more of the following operations: filtration, filtration under vacuum, decantation, and centrifugation, or other suitable techniques as known to a person skilled in the art. Preferably, step (d) is carried out by filtration of the microspheres thus obtained followed by a washing step; preferably with water, ethanol, acetone and mixture thereof. In an embodiment, step (d) further comprises drying the isolated microspheres; preferably the cocrystal is dried at room temperature, preferably under vacuum conditions. Generally, the vacuum involves a pressure comprised from 0.5 mbar to 3 mbar. In an embodiment, step (d) further comprises sieving the isolated microspheres and collected.

In an embodiment, the composition of the invention comprises sol-gel silica microspheres obtainable by a process wherein steps (a), (b), optionally (b'), (c) and (d) are performed at room temperature. The term "room temperature" refers to a temperature from 15°C to 30 °C.

In an embodiment, the composition of the present invention further comprises:
one or more excipients or carriers; a therapeutically effective amount of one or more pharmaceutical active ingredients; or one or more excipients or carriers and a therapeutically effective amount of one or more pharmaceutical active ingredients.

In an embodiment, the composition of the present invention further comprises one or more excipients or carriers. The appropriate excipients and/or carriers, and their amounts, can readily be determined by those skilled in the art according to the type of formulation being prepared. Examples of appropriate excipients and/or carriers include, but is not limited to, fillers, radiocontrast agent and plasticizer.

The terms "filler" and "diluent" have the same meaning and are used interchangeably. They refer to excipients or carriers that fill out the size of a composition, making it practical to produce and convenient for the consumer to use. Materials commonly used as filler include, but without limitation, zinc oxide, calcium hydroxide, or a mixture thereof.

The terms "radiocontrast agent" or "radioopaque agent" have the same meaning and are used interchangeable. They refer to substances used to enhance the visibility of internal structures in X-ray-based imaging techniques such as computed tomography (contrast CT), projectional radiography, and fluoroscopy. They absorb external X-rays, resulting in decreased exposure on the X-ray detector. Examples of appropriate radiocontrast agents are typically iodine, barium-sulphate, bismuth oxide, titanium oxide, tantalum oxide, cerium oxide tin oxide, zirconium oxide compounds and radiopaque glasses containing tantalum, barium and strontium. and mixtures thereof. Typically, the amount of the radioopaque agents is from 9 to 13% by weight of the composition.

The term "plasticizer" refers to a substance that is habitually used to improve the physical and mechanical properties of a material, particularly a polymer, improving properties such as elastic elongation, deformability, tensile strength, flexibility. Examples of appropriate plasticizers include, but without limitation, wax, resins, polyethylenglycol and mixture thereof. In an embodiment, the plasticized in wax. Examples of appropriate wax include, but without limitatin, to beeswax, carnauba wax, candelilla wax, ouricury, Japan wax, cork fiber wax, cane wax sugar, paraffin wax, lignite wax, microcrystalline waxes, lanolin wax, mountain wax, ozokerites and hydrogenated oils, such as hydrogenated jojoba oil, as well as waxes of origin synthetic, for example polyethylene waxes derived from the polymerization or copolymerization of ethylene, waxes obtained by Fischer-Tropsch synthesis, esters of fatty acids and glycerides that are solid at 40 ° C, for example at a temperature above 55 ° C, silicone waxes, such as alkyl- and alkoxypoly (di) methylsiloxanes and / or esters of poly (di) methylsiloxane that are solid at 40 ° C, for example at a temperature higher than 55 ° C. Typically, the amount of the plasticizers is from 1 to 4% by weight of the composition.

In an embodiment, the composition of the present invention further comprises one or more fillers, one or more radioopaque agent and one or more plasticizer. In an embodiment, the composition of the present invention further comprises zinc oxide as the filler, barium sulphate as radioopaque agent and wax as plasticizer.

In an embodiment, the composition of the present invention is a "pharmaceutical composition" further comprising a "therapeutically effective amount of one or more an active pharmaceutical active ingredient" together with one or more "pharmaceutically acceptable excipients or carriers". The term "pharmaceutical composition" refers to a composition suitable for use in the pharmaceutical technology with medical use. The term "therapeutically effective amount of a pharmaceutical active ingredient" as used herein, refers to the amount of a pharmaceutical active ingredient that, when administered, is sufficient to prevent development of, or alleviate to some extent, one or more of the symptoms of the disease which is addressed. The dose of the active pharmaceutical ingredient administered according to this invention will of course be determined by the particular circumstances surrounding the case, including the particular patient being treated, the particular condition being treated, and the similar considerations. For the purpose of the invention the terms "pharmaceutical active ingredient" and "API" have the same meaning and are used interchangeable. They refer to an ingredient in the composition which causes the direct (therapeutically) effect on the treatment, cure or alleviation of a disease or condition. Examples of pharmaceutical active ingredients include, but is not limited to, antibacterial, anti-tumoral or pro-angiogenesis drugs/ions release to hard tissue regeneration and repair. The term "pharmaceutically acceptable excipients or carriers" refers to that excipients or carriers suitable for use in the pharmaceutical technology for preparing compositions with medical use. When the composition comprises one or more an active pharmaceutical active ingredient, those are forming part of the sol-gel silica microspheres as defined above. It is specially advantegeous because the phamaceutical ingredients can be locally released in a sustained manner. The encapsulation of the pharmaceutical active ingredient within the silica microspheres provides a near zero order kinetic release of the encapsulated pharmaceutical active ingredient, enabling a sustained release compared to the coating or soaking of the pharmaceutical active ingredient on the surfce of the microspheres. Furthermore, the encapsulation within the silica microspheres protects the pharmaceutical active ingredients from the harsh enviornment, mainly organic solvents and temperature, used during the processing of the gutta-percha. The local release of pharmaceutical active ingredients can mitigate adverse inflammatory reactions, inflammation and even stimulate a pro-regenerative singalling within the surrounding tissues. In an embodiment, the composition of the present invention further comprises one or more excipients or carriers and a therapeutically effective amount of one or more pharmaceutical active ingredients. All the embodiments disclosed above and below for the excipients or carriers, and for the pharmaceutical active ingredients also apply here.

In an embodiment, the composition of the present invention comprises:

From 15 to 20 % by weight of a polyisoprene-based matrix;
From 1 to 30 % by weight sol-gel silica microspheres;

From 0% to 72% by weight of one or more excipients or carriers;
From 0% to 1% by weight of one or more pharmaceutical active ingredients; and
being the sum of the components up to 100 % by weigth.

In an embodiment, the composition of the invention has a yield stress from 3 MPa to 8 MPa. In an embodiment, the composition of the invention has a yield stress from 3 MPa to 6 MPa. In an embodiment, the composition of the invention has a yield stress about 6 MPa.

In an embodiment, the composition of the invention has an elastic modulus from 150 MPa to 250 MPa. In an embodiment, the composition of the invention has an elastic modulus from 150 MPa to 250 MPa. In an embodiment, the composition of the invention has an elastic modulus is about 220 MPa.

In an embodiment, the composition of the invention has a yield stress from 3 MPa to 8 MPa and an elastic modulus from 150 MPa to 250 MPa. In an embodiment, the composition of the invention has a yield stress from 3 MPa to 8 MPa and an elastic modulus from 150 MPa to 250 MPa. In an embodiment, the composition of the invention has a yield stress about 6 MPa and an elastic modulus is about 220 MPa.

The second aspect of the invention relates to a process for the preparation of the composition of the first aspect of the invention comprising:

(i) mixing polyisoprene with sol-gel silica microspheres; and optionally, with one or more excipients or carriers; and optionally with one or more solvents

(ii) drying the composition obtained in step (i); and

(iii) optionally, molding the composition obtained in step (ii); and drying the composition thus obtained.

This process is a simple and cheap process which is performed under mild conditions. It is advantegeous because it allows dispesing the sol-gel silica microspheres homogeneously distributed on the polyisoprene under a mild conditions without having agregates of microspheres in the surface of the composition.

All embodiments disclosed above for the polyisoprene, sol-gel silica microspheres, excipients or carriers, and pharmaceutical active ingredients of the composicion of the first aspect of the invention, also apply here for the preparation process of the second aspect of the invention.

In an embodiment, step (i) of the process for the preparation of the composition of the invention is performed by sonication. In an embodiment, step (i) of the process for the preparation of the composition of the invention is performed by sonication for 1 to 60 min, particularly, from 5 to 30min; particualrly from 10 to 20 min; particularly about 15min. In an embodiment, step (i) of the process for the preparation of the composition of the invention comprising mixing from 1 to 30% by weight of the sol-gel silica microespheres in relation to the weight of the polyisoprene; particularly from 5 to 20% by weight; particularly from 8 to 15% by weight; particulalry about 10% by weight. In an embodiment, step (i) of the process for the preparation of the composition of the invention is performed at room temperature.

In an embodiment, step (i) of the process for the preparation of the composition of the invention is performed by stirring at a temperature from 30 to 60 °C. In an embodiment, wherein when the composition comprises pharmaceutical active ingredients, then step (i) is performed by stirring at a temperature from 30 to 40°C. In an embodiment, wherein when the composition does not comprises pharmaceutical active ingredients, then step (i) is performed by stirring at a temperature from 30 to 60°C.

In an embodiment, wherein step (i) of the process for the preparation of the composition of the invention is performed in the presence of one or mor solvents, then step (i) is performed by stirring or alternatively by sonication, at a temperature from room temperature to 40 °C.

In an embodiment, the drying steps are performed under the needed period of time since having a weight variation of the composition thus obtained equal to or lower than 5% by weight. Typically, the drying steps are performed at a temperature from room temperature to 35°C. In an embodiment, drying step (ii) is performed for a period from 2 days to 21 days; particularly about 7 days. In an embodiment, drying step of step (iii) is performed for a period from 2 days to 21 days until dryness; particulalry for 21 days.

In an embodiment, the moulded step (iii) is performed in a mould, particularly in a teflon mould. In an embodiment, the moulded step (iii) is performed under vacuum conditions.

In an embodiment, the composition of the invention further comprises one or more excipients or carriers; then, the process further comprises a previous step (iv') before step (i) of mixing polyisoprene with one or more excipients or carriers; and optinally one or more solvents; and the mixture thus obtained is mixed with the sol-gel silica microspheres. In an embodiment, step (iv') is performed by mixing; typically by sonication or stirring as defined above.

In an embodiment, when step (i) comprises the use of one or more solvent, the process further comprises a previous step (v) before step (i) or alternatively before setp (iv) which comprises mixing the isoprene with the one or more solvents. In an embodiment, step (i) of the process of the invention comprises the use of one or more solvents selected from the group consisting of eucalyptol, xylol, chloroform, orange oil, xylene and mixture thereof.

The third aspect of the invention relates the composition of the invention for use in dentistry. In an embodiment, the composition of the invention is useful in dentistry for obturating or filling the root canal in endodontic therapy. In an embodiment, the composition of the present invention is useful as a filler, particularly for sealing a tooth root canal.

The fourth aspect of the invention relates to a process for filling a root canal comprising applying a composition of the present invention in the root canal of a tooth.

In an embodiment, the process for filling a root canal comprises:

(I) preparing the root canal in the tooth to be filled; and
(II) filling the root canal with a composition of the present invention, and
(III) compacting and/or hardening the composition into the root canal.

Typically, step (I) comprises mechanical preparation and instrumentation by endodontic files in order to extract the pulp tissue and to widen the root canals; and chemical disinfection (for instance with sodium hypochlorite solution) in order to clean and eliminate the rests of dentin debris and pulp tissue inside the canals.

In an embodiment, step (II) of the fourth aspect of the invention comprises filling the root canal with a composition of the present invention.

In an embodiment, step (III) of the fourth aspect of the invention comprises filling the root canal with a composition of the present invention. Commonly, the compacting may be performed by any compacting technique known in the state of the art, for instance cold lateral compaction, warm compaction: vertical or lateral, continuous wave compaction, thermo-plasticized GP injection or carrier-based GP.

The process for filling a root canal of the present invention which comprises the use of the composition as self-adhesive filler allows reducing the formation of gaps between the composition and the surface of the tooth and the subsequent re-infection.

All embodiments disclosed above in relation to the composition of the first aspect of the present invention also applies for its use of the third aspect of the invention and the process of the fourth aspect of the invention.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

## Examples

### 1. Materials

### 1.1. Sol-gel Microspheres (SiMS)

The sol-gel silica microspheres (SiMS) were prepared by a sol-gel process at room temperature as disclosed below.

Process

Tetraethyl orthosilicate (TEOS, $C_8H_{20}O_4Si$, 98%, Sigma-Aldrich) (5 mL) was mixed with 0.1 M HCl (1,2 ml), with the addition of deionized water to form an acid catalyzed sol. It was stirred at 300 rpm and once the sol was obtained, 0.08 M ammonium hydroxide ($NH_4OH$, 28.0% $NH_3$ in water, 99.99% metal basis, Sigma-Aldrich) was added dropwise to the sol with agitation and the pH was adjusted to 5-5.5. 6.25 mL of the sol was then added dropwise to 125 mL of olive oil, and was stirred at 250 rpm to allow gelation.

Gelled microspheres were gathered after precipitation at the bottom of the flask, vacuum filtered, rinsed with water, ethanol and acetone, and left overnight to dry. After drying, sol-gel silica microspheres were sieved and the ones at size diameter of 20-100 $\mu$m were collected.

### 1.2. Guttapercha Composite (SiMS-GP)

Preparation of Gluttapercha (GP)

Commercial GP (comm-GP) cones are composed of 20% trans-polyisoprene (TPI), 66% zinc oxide ($ZnO_2$), 11% barium sulphate ($BaSO_4$) and 3% wax. The GP used in the present invention has the same composition as the commerical GP, but it was prepared following the process disclosed below.

Thus, GP was prepared by mixing TPI, $ZnO_2$, $BaSO_4$ and wax (Sigma-Aldrich). Firstly, 6.75 g of TPI was dissolved in 50 mL of chloroform. Then, 22.4 g of $ZnO_2$, 3.74 g of $BaSO_4$ and 1.02 g of wax were added to the polyisoprene solution firstly prepared followed by 15 minutes of sonication after the addition of each component.

Preparation of the guttapercha composition (SiMS-GP)(Composition Ex.1)

The SiMS-GP was prepared by adding 10% by weight of the sol-gel silica microspheres in relation to the weight of the GP (previously prepared) to the guttapercha (previously prepared).

Thus, 3.8 g (10%) of SiMS were added to the guttapercha obtained above and sonicated into the mix for 15 minutes to disperse any SiMS aggregates. After that, they were left to dry first for one week before being moulded into teflon cylinders. After obtaining the desired form, the samples were left drying again for 3 weeks at room temperature in the fume hood in order to let the solvent evaporate.

## 2. Sample characterization

### 2.1. Determination of the distribution of the sol-gel silica microspheres (SiMS)

Morphology and micro-structure of the composition Ex.1 of the present invention were analysis by SEM and optical microscope. The aim of this assay is the observation of the homogeneously distribution of the sol-gel silica microspheres within composite.

Results

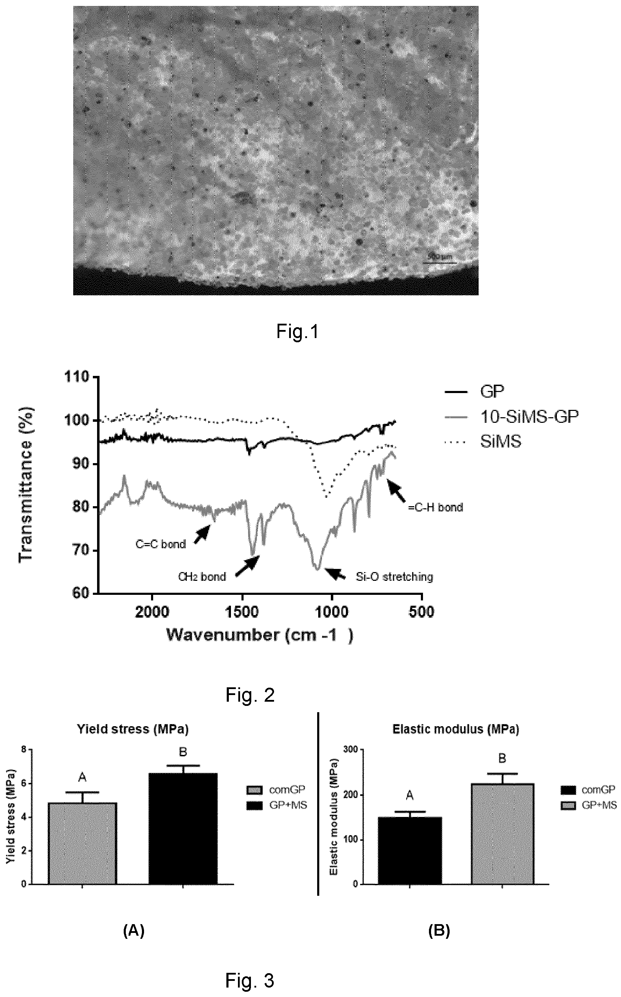

As it is observed in Fig.1, in comparison with the GP doped with melted nanoparticles which are agregated in the surface of the GP; the sol-gel silica microspheres (SiMs) were homogeneously distributed within the whole polyisoprene-based matrix, which means in both the surface and also within the internal part of the cones.

### 2.2. Chemical Properties

Fourier Transformed Infrared Spectroscopy (FTIR) was used for the analysis of the chemical properties of the composition Ex.1 of the present invention, comparative GP, and comparative sol-gel silica microspheres. The aim of this assay is verify if there were any chemical interactions between the components of the composition of the invention.

The assay consisted on first obtaining a disk from each sample prepared above; then the disk was placed in the spectrometer (Agilent Cary 630 FTIR) and the measurement was done. The infrared spectrum is detected at a wave number range of 650 to 4000 cm-1 with a resolution of 4 cm-1. The spectrums were done 6 times and an average spectrum was obtained. A blank with no sample was measured in order to have the base line. The spectrums were obtained and analysed using the MicroLab Lite software.

Results

As it is shown in Fig.2, the significant shift of the SiMS containing polyisoprene-based matrix of the present invention in comparison with the pristine polyisoprene-based matrix falling outside of the scope of protection of the present invention, which means electrostatic interactions between the SiMS and polyisoprene-based matrix are present.

## 3. Mechanical properties

Universal testing machine (Galdabini) were used for the analysis of the mechanical properties of the composition Ex.1 of the present invention and commercial GP.

The tested samples were prepared by heating them at 50°C and the slurries were introduced into teflon cylinder moulds of 12 mm height and 6 mm diameter. Then, each sample were left dry for 3 weeks at room temperature in order to assure that the chloroform has evaporated after proceeding with the test. A standard number of ten cylinders were prepared for each tested sample and used for compression testing in Universal Testing Machine (Galdabini) at a crosshead spead of 1 mm/min. The force was axially applied.

Yield strain, yield stress and elastic modulus of each tested sample were calculated. The maximum force applied was calculated versus the maximum displacement. These units were given in Newtons and mm, respectively.

The "yield stress" or "yield strength" is a material property which is the stress ($\sigma$) corresponding to the yield point at which the material begins to deform plastically. Particularly, the yield point is the point on a stress-strain curve that indicates the limit of elastic behavior and the beginning of plastic behavior. Below the yield point, a material will deform elastically and will return to its original shape when the applied stress is removed. Once the yield point is passed, some fraction of the deformation will be permanent and non-reversible and is known as plastic deformation.

The force (F) was transformed into stress ($\sigma$), by dividing the force (F) by the area (A):

$$\sigma = F/A \ [MPa]$$

Then, strain was obtainable by calculating the change of the length divided by the original length (l0)of the material:

$$\varepsilon = (l1 - l0)/l0 \ [\%]$$

where l1 is length after elongation and l0 is original length (mm).

The "Elastic modulus" or "modulus of elasticity" is a quantity that measures an object or substance's resistance to being deformed elastically (i.e., non-permanently) when a stress is applied to it. The elastic modulus values were obtained from the slope of the straightline portion of a stress ($\sigma$) strain ($\varepsilon$) curve. Therefore, the modulus is the change in stress divided by the change in strain, calculated by the next equation:

$$Modulus = (\sigma2 - \sigma1)/(\varepsilon2 - \varepsilon1)$$

where stress ($\sigma$) is force (F) divided by the specimen's cross-sectional area (A) and strain ($\varepsilon$) is the change in length of the material divided by the material's original gauge length.

Results

As it is shown in Fig 3, the composition of the present invention has a higher yield stress (cf. Fig 3A) and a higher elastic modulus (cf. Fig 3B) than the commercial guttapercha (commGP) falling outside the scope of the present invention. The enhanced mechanical properties of the composition of the invention is advantegeous because allows avoiding the compression (reduction of size) or expansion (increase of size) of the composition of the invention when it is inside the root canal of the teeth. This maintaince of the size avoid the formation of gaps between the tooth and the composite and the loss of the composite and/or the re-infection of the tooth by the re-entrance of bacteriae.

**4. In vitro apatite forming ability**

The aim of this assay is the measuring of the sealing ability of the composition of the present invention, which means an optimal bonding to dentine tissue (which has a very similar composition to bone tissue) of the composition Ex.1 of the present invention and commercial glutapercha.

The assay involves immersing the tested compositions in simulated body fluid (SBF) solution to study the apatite-forming ability on its surface. This assay is extensively used to evaluate the bone-bonding ability of a material because SBF solution has ion concentrations nearly equal to those of human blood plasma. We have prepared SBF solution following the process disclosed in Kokubo, T. & Takadama, H. et al. "How useful is SBF in predicting in vivo bone bioactivity?". Biomaterials, 2006, vol. 27, pp. 2907-2915 (all components were obtained from Sigma-Aldrich).

First of all, all containers and utensils were washed with diluted acid, neutral soap and deionized water. After that, the reagents presented in Table below were mixed with 500 ml of deionized water in the order indicated and waiting for it to dissolve before adding the next reagent. SBF solution was buffered at pH 7.4 with the addition of 50 mM Tris and 45 mM HCl (the last reagents of the Table), and the temperature was kept constant at 37°C. Finally, deionized water was added to complete 1 L of solution.

The below table discloses the order and amounts (expressed in grams) of reagents for preparing 1000 ml of SBF.

| Order | Reagent | Amount |
|---|---|---|
| 1 | Sodium chloride (NaCl) | 7,996 g |
| 2 | Sodium hydrogen carbonate (NaHCO$_3$, | 0.350 g |

(continued)

| Order | Reagent | Amount |
|---|---|---|
| | | |
| 3 | Potassium chloride (KCl) | 0.224 g |
| 4 | Di-potassium hydrogen phosphate trihydrate ($K_2HPO_4 \cdot 3H_2O$) | 0.228 g |
| 5 | Magnesium chloride hexahydrate ($MgCl2 \cdot 6 H_2O$) | 0.305 g |
| 6 | $1_M$ (mol/l) hydrochloric acid ($1.0_M$-HCl) | 37,5 ml |
| 7 | Calcium chloride ($CaCl_2$) | 0.278 g |
| 8 | Sodium sulfate ($Na_2SO_4$) | 0.074 g |
| 9 | Tris-hydroxymethyl aminomethane Tris | 6.057 g |
| 10 | $1_M$ (mol/l) hydrochloric acid ($1.0_M$-HCl) | 0-5 ml |

The composition of the present invention Ex.1 and the GP were prepared as previously explained at size of 3 mm height and 6 mm diameter. Moreover, one hundred milligrams of microspheres with diameters between 20 and 100 $\mu$m were collected to be used as control sample. All samples were incubated per triplicate in 50 ml of SBF solution for 14 and 21 days. The SBF solution volume needed was calculated by the next equation:

$$V = A / 10$$

where V is the volume of the necessary medium for each sample and A is the apparently superficial area of the sample.

After the incubation period, the samples were collected and separated from the solution, gently rinsed with deionized water to remove any debris and finally they were left to dry for 24 hours. The samples were then mounted on aluminium stubs, carbon-sputtered and examined with a Scanning Electron Microscopy (SEM) and Energy Dispersive x-ray Spectroscopy (EDS) (JEOL JSM-7001F).

EDS is used to obtain information about which elements are present in the sample analysed. This technique measures the X-ray energy emitted by the material, giving information about its composition. EDS was used in order to assess the presence of calcium phosphate and the different elements of the materials.

Results

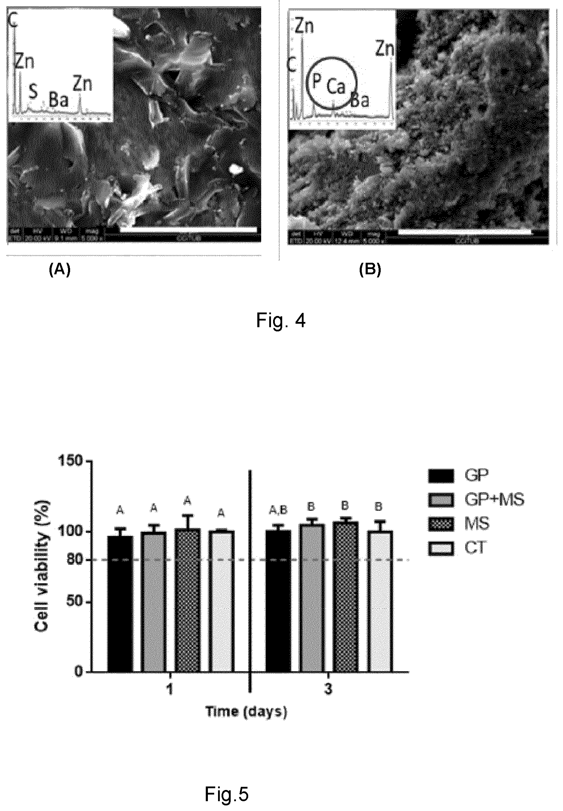

As it is shown in Fig. 4, the composition of the present invention allows the formation of apatite both in the surface of the composite and inside the composite (by the diffusion of the fisiological media inside the porous polyisoprene based matrix). This specific capability of apatite formation enhances the mechanical properties of the composition (cf. section above) and improve its in vivo behaviour for restoring bone or bone-related tissues.

**5. Cell proliferation assay**

Indirect cell proliferation assay was performed in order to assess the biocompatibility of the composition Ex.1 of the present invention, comparative GP, comparative sol-gel silica microspheres and comparative control.

**5.1. Material preparation**

The material samples were prepared as follows: first of all, the ratio of material surface area to medium volume was set approximately 1,25 $cm^2$/mL in accordance with the guidelines of the International Organization for Standardization 10993-12. Following this standard, the materials were shaped into 7 mm height and 6 mm diameter cylinders. 30 mg of sol-gel silica microspheres (SiMS) were used as control.

All samples were sterilized immersing them in ethanol solution for 3 times during 15 minutes and then in PBS solution during the same time. After that, ultraviolet irradiation was turned on during 30 minutes on each side of the samples and stored in the incubator at 37°C. All samples were used in triplicate.

The eluates of the different materials were obtained in sterile conditions, using basal culture medium as extraction vehicle. The extraction process was as follows: first, the materials were maintained in the basal medium for 24 hours at 37°C in a humid atmosphere containing 5% $CO_2$. The conditioned medium extracted was filtered with sterile filters of 0.22 $\mu$m diameter pores (Thermo Fisher), and several dilutions of basal medium with conditioned medium were prepared. Basal medium without materials served as a control.

### 5.2. Cell culture

Rat mesenchymal stem cells (rMSCs) were obtained in accordance with an ethics approved protocol from the Research Committee from the Universitat Internacional de Catalunya. Cells were isolated from 5 week old Sprague-Dawley male rat and were analysed to confirm that they were optimal to be used in the next assay. Cells at passage 3 were used in order to assess the cell proliferation in an indirect experiment with the materials. The cells were cultured in DMEM with 4,5 g/L glucose supplemented with 20% fetal bovine serum (FBS), 1% L-glutamine (GlutaMax), 1% penicillin/streptomycin (Sigma-Aldrich), and incubated at 37°C in a humidified atmosphere of 5% CO2 in air. The culture medium was exchanged every second day. Upon confluence, the cells were detached with a minimum amount of Accutase (Sigma-Aldrich). The cells were then seeded in 24-well plates at a density of 1 x 104 cells per well with 500 $\mu$L of basal medium and allowed to attach for 24 hours. After that, the cells medium was changed for the conditioned medium of the materials at different dilutions (1, 1:5, 1:10 and 1:50). Cell-only with basal medium served as a control.

### 5.3. Indirect cell proliferation assay

Cell viability was evaluated at 1 and 3 days using the commercial available Cell Counting Kit (CCK-8) (Sigma-Aldrich). This assay uses a water-soluble product known as WST-8 that is reduced by cells and generates a yellow-coloured product (formazan). This metabolized product is soluble in the culture medium. For that purpose, at each time point, the samples medium was changed for fresh one and added a 10% of CCK-8 solution to the cells. Then the plates were left in the incubator at 37°C for 3 hours for the reaction to take place. Aliquots of each sample were placed on 96 well-plate with a sample volume of 100 $\mu$L. A calibration curve with decreasing concentrations of cells was created to express results in cell number. The CCK-8 activity was then determined spectrophotometrically with a multi-detection microplate reader (Bio-Tek Synergy HT) by measuring absorbance at 450 nm.

Results:

As it is demonstrated in Fig. 5, the cell viability expressed in percentage is comparable with the guttapercha and it is concluded that the composite of the present invention is biocompatible with tissues, and particularly with the tooth.

### 6. Sealing ability assay

This assay consists on performing an in vitro endodontic treatment following exactly the same steps as performed in the clinic. The aim of the assay is the assessment and comparison between different root canal filling materials or techniques.

### 6.1. On dental training blocks

Samples:

> Sample 1: commercial GP cones
> Sample 2: composition Ex.1 of the present invention cones (10-SiMS-GP manually-fabricated cones as disclosed above)

Assay:

The samples were heated with a blowtorch and manually shaped with a metal spatula until a cone-shape similar to comGP was obtained to be introduced in the dental training blocks. These blocks used were methacrylate endotrain beakers with a simulated root canal longitude size of 14 mm x 3 mm diameter (Dentsply Sirona).
The mechanical preparation of the simulated root canal was done with the use of Niquel Titanium-made rotary Protaper files (Maillefer) with different tapers and diameters in order to wide and shape the canal. An automated torque control motor was used with a speed set to 300 rpm for all rotary files. The torque was set to 2.0 (N/cm). Between each rotary file instrumentation, an irrigation with 3 ml of 5% sodium hypochlorite solution was done in order to eliminate the debris inside the canal and avoiding its blocking 30.
The canals were then dried with paper points. Then, they were randomly divided into 2 groups of 3 specimens each and the cone-shaped materials were introduced in the canals to perform the canal filling:

Group 1: commercial GP cones (sample 1)
Group 2: 10-SiMS-GP manually-fabricated cones (sample 2)

Excess GP cone was seared off from the canal orifice using a heated endodontic metal instrument. After the filling procedure, the beakers were stored at room temperature for 24 hours and 10 μL of blue dying was applied on the top orifice of the beakers in order to assess the leakage. They were left for 7 days and photographs were taken to compare the filtration of the dye on the different samples.

Results:

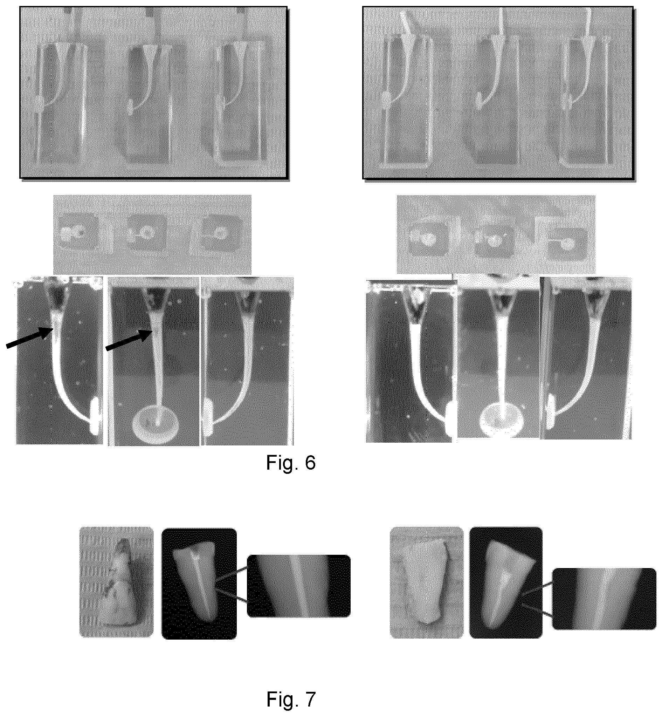

As it is shown in Fig. 6, the commercial GP cones of the state of the art present higher infiltration of dye compared to siMS-GP of the present invention, showing better sealing capability. It is advantegeous because the reduction of infiltratoin reduces the amount of reinfections and the loss of the filling material outside the root canal.

## 6.2. On extracted teeth

Samples:

Sample 1: GP self-fabricated cones
Sample 2: composition Ex.1 of the present invention cones (10-SiMS-GP manually-fabricated cones as disclosed above)

Assay:

Patient-derived teeth samples were extracted for orthodontic or periodontal reasons at the UIC dental clinic and they were obtained in accordance with an approved protocol by our institution.
Teeth with caries, multiple canals, root resorption, open apex or root curvatures were excluded so that the level of expertise did not play a factor in the success of the root canal treatment. Six single-root incisors were chosen for the experiment. The teeth were immersed in a disinfection solution during one week and then in saline solution. They were decoronated using diamond disk at the cement-enamel junction uniformly. The root canal access was prepared using an endo access bur. After that, an endo Z bur was used to create and refine the proper walls of the chamber and the working length was determined using a 010 K-file.
Finally, the mechanical preparation and root canal filling protocol was the same as described above. In this case, we opted to compare sample 1 (composition Ex.1 of the present invention cones) of the present invention as with self-fabricated GP (disclosed above) falling outside the scope of the invention in order to observe if there were differences with the same fabricated material at the same conditions, with the only difference of adding sol-gel silica microspheres (SiMS) or not to the polyisoprene material (GP).Thus, they were divided into 2 groups:

Group 1: GP self-fabricated cones
Group 2: composition Ex.1 of the present invention cones (10-SiMS-GP manually-fabricated cones as disclosed above)

After the filling procedure, a final digital radiograph was taken to assess the quality of the filling. The X-ray tube and digital sensor were settled to allow radiographs to be exposed using the paralleling technique.

Results:

As it is shown in Fig. 7, the siMS-GP cones of the present invention allow an adecuate filling of extracted teeth comparable to the commerical GP cones falling outside the scope of protection of the present invention.

**Citation List**

1. D. Mohn et al. "composites made of flame-sprayed bioactive glass 45S5 and polymers: bioactivity and inmediate sealing properties", International Endodontic journal, 2010, vol. 43, pages 1037-1046).

2. T. & Takadama, H. et al. "How useful is SBF in predicting in vivo bone bioactivity?". Biomaterials, 2006, vol. 27, pp. 2907-2915.
3. Ross Hallett "Particle size analysis by dynamic light scattering". Food Research International, 1994, vol. 27, issue 2, pp. 195-198.
4. US2008085948
5. JP2016098202
6. US2002037258

**Claims**

1. A root canal filling composition comprising:

   a polyisoprene-based matrix; and
   sol-gel silica microspheres;
   wherein:

   the sol-gel silica microspheres are homogeneously distributed in the polyisoprene-based matrix;
   the polyisoprene-based matrix is selected from the group consisting of trans-polyisoprene, gutapercha, and mixtures thereof; and
   the sol-gel silica microspheres have a diameter from 5 $\mu$m to 500 $\mu$m measured by dynamic laser scattering.

2. The composition according to claim 1, wherein the composition further comprises:

   one or more excipients or carriers;
   a therapeutically effective amount of one or more pharmaceutical active ingredients; or
   one or more excipients or carriers; and a therapeutically effective amount of one or more pharmaceutical active ingredients.

3. The composition according to any of the claims 1 or 2, wherein the polyisoprene-based matrix is gutapercha.

4. The composition according to any of the claims 1-3, wherein the sol-gel silica microspheres have a diameter from 20 $\mu$m to 100 $\mu$m measured by dynamic laser scattering.

5. The composition according to any of the claims 1-4, wherein the sol-gel silica microspheres are obtainable by a process which comprises:

   (a) providing a sol by mixing one or more silicon alkoxides with one or more acid catalysts;
   (b) adjusting the pH of the sol obtained in step (a) from 4 to 5.5;
   (b') optionally, adding the therapeutically effective amount of the one or more pharmaceutical active ingrediant, and optionally stirring until having a homogeneous mixture within the sol;
   (c) adding the sol obtained in step (b) or (b') to an oil phase, and stirring until having the microspheres; and
   (d) optionally, isolating the microspheres obtained in step (c).

6. The composition according to claim 5, wherein the sol-gel silica microspheres are obtainable by a process wherein in step (a) the silicon alkoxide is a compound of formula (I)

$$(OR^1)(OR^2)(OR^3)(OR^4)Si \qquad (I)$$

   wherein:

   each one of $R^1$, $R^2$, $R^3$ and $R^4$ are independently selected from the group consisting of substituted or unsubstituted $(C_1$-$C_{14})$alkyl, $(C_5$-$C_6)$aryl, $(C_2$-$C_{14})$alkenyl and $(C_2$-$C_{14})$alkynyl group; and
   the silicon alkoxide of formula (I) comprises a silicon central atom that forms covalent bonds with oxygen-carbon and has from 4 to 56 carbon atoms.

7. The composition according to any of the claims 5 or 6, wherein the sol-gel silica microspheres are obtainable by a process wherein in step (a) the silicon alkoxides is tetraethyl orthosilicate.

8. The composition according to any of the claims 5-7, wherein the sol-gel silica microspheres are obtainable by a process wherein in step (a) the acid catalyst is an inorganic acid selected from the group consisting of $H_2SO_4$, HCl, $HNO_3$, and a mixture thereof.

9. The composition according to any of the claims 5-8, wherein the sol-gel silica microspheres are obtainable by a process wherein:

> step (b) is performed by the addition of an one or more pH adjusting agents selected from ammonium hydroxide, alkaline or alkaline earth metal hydroxide, and a mixture thereof;
> particularly selected from the group consisting of sodium hydroxide, postasium hydroxide, calcium hydroxide, ammonium hydroxide and a mixture thereof; and
> in step (c), the oil phase comprises one or more oils selected from the group consisting of olive oil, sunflower oil, vaseline ol, palm oil and a mixture thereof; particularly olive oil.

10. The composition according to any of the claims 1-9, which has a yield stress ($\sigma$) from 3 to 8 MPa measured by performing a compression Test in the Universal Testing Machine at a crosshead spead of 1 mm/min applying a axial force and calculting the value by the following equation:

$$\sigma = F/A \text{ [MPa]}$$

being F the force and A the specimen's cross-sectional area.

11. The composition according to any of the claims 1-10, which has an elastic modulus from 150 to 250 MPa measured by performing a compression Test in the Universal Testing Machine at a crosshead spead of 1 mm/min applying a axial force and calculting the value by the following equation:

$$\text{Modulus} = (\sigma2 - \sigma1) / (\varepsilon2 - \varepsilon1) \text{ MPa}$$

Wherein:

> $\sigma$ is the yield stress value as defined in claim 10 and
> $\varepsilon$ is the strain calculated by the following equation:

$$\varepsilon = (l1 - l0) / l0 \text{ [\%]}$$

being l1 the length after elongation and l0 the original length expressed in mm.

12. A process for the preparation of the composition as defined in any of the claims 1-11, comprising:

> (i) mixing polyisoprene with sol-gel silica microspheres; and optionally, with one or more excipients or carriers; and optionally one or more solvents;
> (ii) drying the composition obtained in step (i); and
> (iii) optionally, molding the composition obtained in step (ii); and drying the composition thus obtained.

13. The composition according to any of the claims 1-11, for use in dentistry.

14. The composition according to claim 13, for use as root canal filler.

**Patentansprüche**

1. Eine Wurzelkanalfüllungszusammensetzung umfassend:

> eine Matrix auf Polyisoprenbasis; und
> Sol-Gel-Kieselsäure-Mikrosphären;
> wobei:

die Sol-Gel-Kieselsäure-Mikrosphären homogen in der Matrix auf Polyisoprenbasis verteilt sind;
die Matrix auf Polyisoprenbasis ausgewählt ist aus der Gruppe bestehend aus Trans-Polyisopren, Guttapercha und Mischungen davon; und
die Sol-Gel-Kieselsäure-Mikrosphären einen Durchmesser von 5 μm bis 500 μm, gemessen durch dynamische Laserstreuung, haben.

2. Die Zusammensetzung nach Anspruch 1, wobei die Zusammensetzung ferner Folgendes umfasst:

einen oder mehrere Hilfsstoffe oder Trägersubstanzen;
eine therapeutisch wirksame Menge eines oder mehrerer pharmazeutischer Wirkstoffe;
oder eines oder mehrerer Hilfsstoffe oder Trägersubstanzen; und eine therapeutisch wirksame Menge eines oder mehrerer pharmazeutischer Wirkstoffe.

3. Die Zusammensetzung nach einem der Ansprüche 1 oder 2, wobei die Matrix auf Polyisoprenbasis Guttapercha ist.

4. Die Zusammensetzung nach einem der Ansprüche 1 bis 3, wobei die Sol-Gel-Kieselsäure-Mikrosphären einen Durchmesser von 20 μm bis 100 μm, gemessen durch dynamische Laserstreuung, haben.

5. Die Zusammensetzung nach einem der Ansprüche 1 bis 4, wobei die Sol-Gel-Kieselsäure-Mikrosphären durch ein Verfahren erhalten werden können, das Folgendes umfasst:

(a) bereitstellen eines Sols durch Mischen eines oder mehrerer Siliziumalkoholat mit einem oder mehreren Säurekatalysatoren;
(b) einstellen des pH-Werts des in Schritt (a) erhaltenen Sols von 4 auf 5,5;
(b') wahlweise, Zugeben der therapeutisch wirksamen Menge des einen oder der mehreren pharmazeutischen Wirkstoffe und wahlweise Rühren, bis eine homogene Mischung innerhalb des Sols vorliegt;
(c) Zugeben des in Schritt (b) oder (b') erhaltenen Sols zu einer Ölphase und Rühren, bis die Mikrosphären vorliegen; und
(d) wahlweise, Isolieren der in Schritt (c) erhaltenen Mikrosphären.

6. Die Zusammensetzung nach Anspruch 5, wobei die Sol-Gel-Kieselsäure-Mikrosphären durch ein Verfahren erhalten werden können, bei dem in Schritt (a) das Siliciumalkoholat eine Verbindung der Formel (I) ist

$$(OR^1)(OR^2)(OR^3)(OR^4)Si \qquad (I)$$

wobei:

jedes von $R^1$, $R^2$, $R^3$ und $R^4$ unabhängig voneinander ausgewählt ist aus der Gruppe bestehend aus substituierter oder unsubstituierter $(C_1\text{-}C_{14})$-Alkyl-, $(C_5\text{-}C_6)$-Aryl-, $(C_2\text{-}C_{14})$-Alkenyl- und $(C_2\text{-}C_{14})$-Alkinylgruppe; und
das Siliciumalkoholat der Formel (I) ein Silicium-Zentralatom umfasst, das mit Sauerstoff-Kohlenstoff kovalente Bindungen bildet und von 4 bis 56 Kohlenstoffatome hat.

7. Die Zusammensetzung nach einem der Ansprüche 5 oder 6, wobei die Sol-Gel-Kieselsäure-Mikrosphären durch ein Verfahren erhalten werden können, bei dem in Schritt (a) die Siliciumalkoholate Tetraethylorthosilicat ist.

8. Die Zusammensetzung nach einem der Ansprüche 5 bis 7, wobei die Sol-Gel-Kieselsäure-Mikrosphären durch ein Verfahren erhalten werden können, bei dem in Schritt (a) der Säurekatalysator eine anorganische Säure ist, die aus der Gruppe ausgewählt ist, die aus $H_2SO_4$, HCl, $HNO_3$ und einer Mischung davon besteht.

9. Die Zusammensetzung nach einem der Ansprüche 5 bis 8, wobei die Sol-Gel-Kieselsäure-Mikrosphären durch ein Verfahren erhalten werden können, bei dem:

der Schritt (b) durch die Zugabe eines oder mehrerer pH-Einstellungsmittel durchgeführt wird, die ausgewählt sind aus Ammoniumhydroxid, Alkali- oder
Erdalkalimetallhydroxid, und einer Mischung davon; insbesondere ausgewählt aus der Gruppe bestehend aus Natriumhydroxid, Kaliumhydroxid, Calciumhydroxid, Ammoniumhydroxid und einer Mischung davon; und
in Schritt (c), die Ölphase ein oder mehrere Öle umfasst, die aus der Gruppe ausgewählt sind, die aus Olivenöl, Sonnenblumenöl, Vaselineöl, Palmöl und einer Mischung davon besteht; insbesondere Olivenöl.

10. Die Zusammensetzung nach einem der Ansprüche 1 bis 9, welche eine Streckspannung ($\sigma$) von 3 bis 8 MPa hat, gemessen durch die Durchführung eines Kompressionstests in der Universalprüfmaschine bei einer Kreuzkopfgeschwindigkeit von 1 mm/min unter Anwendung einer Axialkraft und Berechnung des Werts nach der folgenden Gleichung:

$$\sigma = F / A \text{ [MPa]}$$

wobei F die Kraft und A die Querschnittsfläche der Probe ist.

11. Die Zusammensetzung nach einem der Ansprüche 1 bis 10, welche einen Elastizitätsmodul von 150 bis 250 MPa hat, gemessen durch die Durchführung eines Kompressionstests in der Universalprüfmaschine bei einer Kreuzkopfgeschwindigkeit von 1 mm/min unter Anwendung einer Axialkraft und Berechnung des Werts nach der folgenden Gleichung:

$$\text{Modul} = (\sigma2 - \sigma1) / (\varepsilon2 - \varepsilon1) \text{ MPa}$$

Wobei:

$\sigma$ der Streckspannungswert wie in Anspruch 10 definiert ist und
$\varepsilon$ die nach der folgenden Gleichung berechnete Dehnung ist:

$$\varepsilon = (l1 - l0) / l0 \text{ [\%]}$$

wobei l1 die Länge nach der Verlängerung und l0 die ursprüngliche Länge in mm ist.

12. Ein Verfahren zur Herstellung der Zusammensetzung wie in einem der Ansprüche 1 bis 11 definiert, umfassend:

(i) Mischen von Polyisopren mit Sol-Gel-Kieselsäure-Mikrosphären; und wahlweise mit einem oder mehreren Hilfsstoffen oder Trägersubstanzen; und wahlweise einem oder mehreren Lösungsmitteln;
(ii) Trocknen der in Schritt (i) erhaltenen Zusammensetzung; und
(iii) wahlweise, Schmelzen der in Schritt (ii) erhaltenen Zusammensetzung; und Trocknen der so erhaltenen Zusammensetzung.

13. Die Zusammensetzung nach einem der Ansprüche 1 bis 11 zur Verwendung in der Zahnmedizin.

14. Die Zusammensetzung nach Anspruch 13 zur Verwendung als Wurzelkanalfüller.

**Revendications**

1. Une composition d'obturation du canal radiculaire comprenant :

une matrice à base de polyisoprène ; et
des microsphères de silice sol-gel ;
dans laquelle :

les microsphères de silice sol-gel sont réparties de manière homogène dans la matrice à base de polyisoprène ;
la matrice à base de polyisoprène est choisie dans le groupe constitué par le transpolyisoprène, la gutta-percha et des mélanges de ceux-ci ; et
les microsphères de silice sol-gel ont un diamètre de 5 $\mu$m à 500 $\mu$m mesuré par diffusion laser dynamique.

2. La composition selon la revendication 1, dans laquelle la composition comprend en outre :

un ou plusieurs excipients ou véhicules ;
une quantité thérapeutiquement efficace d'un ou de plusieurs ingrédients actifs pharmaceutiques ; ou un ou plusieurs excipients ou véhicules ; et une quantité thérapeutiquement efficace d'un ou de plusieurs ingrédients actifs pharmaceutiques.

3. La composition selon l'une quelconque des revendications 1 ou 2, dans laquelle la matrice à base de polyisoprène est la gutta-percha.

4. La composition selon l'une quelconque des revendications 1 à 3, dans laquelle les microsphères de silice sol-gel ont un diamètre de 20 $\mu$m à 100 $\mu$m mesuré par diffusion laser dynamique.

5. La composition selon l'une quelconque des revendications 1 à 4, dans laquelle les microsphères de silice sol-gel peuvent être obtenues par un procédé qui comprend :

(a) fournir un sol en mélangeant un ou plusieurs alcoxydes de silicium avec un ou plusieurs catalyseurs acides ;
(b) ajuster le pH du sol obtenu à l'étape (a) de 4 à 5,5 ;
(b') facultativement, ajouter la quantité thérapeutiquement efficace du ou des plusieurs ingrédients actifs pharmaceutiques, et facultativement agiter jusqu'à avoir un mélange homogène dans le sol ;
(c) ajouter le sol obtenu à l'étape (b) ou (b') à une phase huileuse, et agiter jusqu'à ce que les microsphères soient présentes ; et
(d) facultativement, isoler les microsphères obtenues à l'étape (c).

6. La composition selon la revendication 5, dans laquelle les microsphères de silice sol-gel peuvent être obtenues par un procédé dans lequel dans l'étape (a) l'alcoxyde de silicium est un composé de formule (I)

$$(OR^1)(OR^2)(OR^3)(OR^4)Si \qquad (I)$$

dans laquelle :

chacun de $R^1$, $R^2$, $R^3$ et $R^4$ sont indépendamment choisis dans le groupe constitué de groupe alkyle en ($C_1$-$C_{14}$), aryle en ($C_5$-$C_6$), alcényle en ($C_2$-$C_{14}$) et alcynyle en ($C_2$-$C_{14}$) substitué ou non substitué ; et
l'alcoxyde de silicium de formule (I) comprend un atome central de silicium qui forme des liaisons covalentes avec l'oxygène-carbone et a de 4 à 56 atomes de carbone.

7. La composition selon l'une quelconque des revendications 5 ou 6, dans laquelle les microsphères de silice sol-gel peuvent être obtenues par un procédé dans lequel, dans l'étape (a), les alcoxydes de silicium est l'orthosilicate de tétraéthyle.

8. La composition selon l'une quelconque des revendications 5 à 7, dans laquelle les microsphères de silice sol-gel peuvent être obtenues par un procédé dans lequel, dans l'étape (a), le catalyseur acide est un acide inorganique choisi dans le groupe constitué par $H_2SO_4$, HCl, $HNO_3$ et un mélange de ceux-ci.

9. La composition selon l'une quelconque des revendications 5 à 8, dans laquelle les microsphères de silice sol-gel peuvent être obtenues par un procédé dans lequel :

l'étape (b) est effectuée par l'addition d'un ou de plusieurs agents d'ajustement du pH choisi(s) parmi l'hydroxyde d'ammonium, un hydroxyde de métal alcalin ou alcalinoterreux, et un mélange de ceux-ci ; en particulier choisi(s) dans le groupe constitué par l'hydroxyde de sodium, l'hydroxyde de potassium, l'hydroxyde de calcium, l'hydroxyde d'ammonium et un mélange de ceux-ci ; et
dans l'étape (c), la phase huileuse comprend une ou plusieurs huiles choisies dans le groupe constitué de l'huile d'olive, de l'huile de tournesol, de l'huile de vaseline, de l'huile de palme et d'un mélange de celles-ci ; en particulier l'huile d'olive.

10. La composition selon l'une quelconque des revendications 1 à 9, qui a une limite d'élasticité ($\sigma$) de 3 à 8 MPa mesurée en effectuant un test de compression dans la machine d'essai universelle à une vitesse de tête transversale de 1 mm/min en appliquant une force axiale et en calculant la valeur avec l'équation suivante :

$$\sigma = F \, / \, A \; [MPa]$$

étant F la force et A la section transversale de l'échantillon.

11. La composition selon l'une quelconque des revendications 1 à 10, qui a un module d'élasticité de 150 à 250 MPa mesuré en effectuant un test de compression dans la machine d'essai universelle à une vitesse de tête transversale de 1 mm/min en appliquant une force axiale et en calculant la valeur avec l'équation suivante :

$$\text{Module} = (\sigma_2 - \sigma_1) / (\varepsilon_2 - \varepsilon_1) \text{ MPa}$$

Dans laquelle:

$\sigma$ est la valeur de la limite d'élasticité telle que définie dans la revendication 10 et
$\varepsilon$ est la déformation calculée par l'équation suivante :

$$\varepsilon = (l_1 - l_0) / l_0 \text{ [%]}$$

étant $l_1$ la longueur après l'allongement et $l_0$ la longueur d'origine exprimée en mm.

12. Un procédé de préparation de la composition telle que définie dans l'une quelconque des revendications 1 à 11, comprenant :

(i) mélanger du polyisoprène avec des microsphères de silice sol-gel ; et facultativement, avec un ou plusieurs excipients ou véhicules ; et facultativement un ou plusieurs solvants ;
(ii) sécher la composition obtenue à l'étape (i) ; et
(iii) facultativement, faire fondre la composition obtenue à l'étape (ii) ; et sécher la composition ainsi obtenue.

13. La composition selon l'une quelconque des revendications 1 à 11, pour une utilisation en dentisterie.

14. La composition selon la revendication 13, pour l'utilisation en tant qu'obturation pour le canal radiculaire.

Fig.1

Fig. 2

(A)                                                    (B)

Fig. 3

(A)                                        (B)

Fig. 4

Fig.5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016098202 B
- US 2002037258 A
- US 2008085948 A

**Non-patent literature cited in the description**

- **D. MOHNET**. composites made of flame-sprayed bioactive glass 45S5 and polymers: bioactivity and inmediate sealing properties. *International Endodontic journal*, 2010, vol. 43, 1037-1046
- **ROSS HALLETT**. Particle size analysis by dynamic light scattering. *Food Research International*, 1994, vol. 27 (2), 195-198
- **KOKUBO, T.** ; **TAKADAMA, H et al.** How useful is SBF in predicting in vivo bone bioactivity?. *Biomaterials*, 2006, vol. 27, 2907-2915
- **T. & TAKADAMA, H. et al.** How useful is SBF in predicting in vivo bone bioactivity?".. *Biomaterials*, 2006, vol. 27, 2907-2915